# EUROPEAN PATENT APPLICATION

(11) **EP 4 362 059 A1**
(43) Date of publication of application: **01.05.2024**
(21) Application number: 23204424.8
(22) Date of filing: 18.10.2023
(51) Int. Cl.: H01J 49/00, G01N 30/72, H01J 49/04

(54) **SYSTEMS AND METHODS FOR PERFORMING DYNAMIC DATA INDEPENDENT ACQUISITION**

(30) Priority: 28.10.2022 US 202217976260
(71) Applicant: Thermo Finnigan LLC, San Jose, CA 95134 (US); University of Washington, Seattle, WA 98105 (US)
(72) Inventor: REMES, Philip M., Livermore (US); YIP, Ping F., Salem (US); MACCOSS, Michael J., Seattle (US); HEIL, Lilian, Seattle (US)
(74) Representative: Boult Wade Tennant LLP

(57) **Abstract**

A system for performing dynamic DIA directs a mass spectrometer to acquire, based on an acquisition schedule that schedules a plurality of acquisition cycles, MS2 spectra of product ions derived from analytes included in a sample as the analytes elute from a separation system. The acquisition schedule specifies, for each acquisition cycle included in the plurality of acquisition cycles, a dynamic precursor m/z range based on an expected elution time of the analytes. The product ions are produced from precursor ions isolated using an isolation window successively positioned throughout the dynamic precursor m/z range during each acquisition cycle. The system detects an elution time shift in the elution time of the analytes as the analytes elute and adjusts the acquisition schedule based on the detected elution time shift.

## Description

This invention was made with government support under Grant Nos. P41 GM103533 and U19 AG065156, awarded by the National Institutes of Health. The government has certain rights in the invention.

### BACKGROUND INFORMATION

A mass spectrometer is a sensitive instrument that may be used to detect, identify, and/or quantify molecules based on the mass-to-charge ratio (m/z) of ions produced from the molecules. A mass spectrometer generally includes an ion source for producing ions from molecules included in a sample, a mass analyzer for separating the ions based on their m/z, and an ion detector for detecting the separated ions. The mass spectrometer may include or be connected to a computer-based software platform that uses data from the ion detector to construct a mass spectrum that shows a relative abundance of each of the detected ions as a function of m/z. The mass spectrum may be used to detect and quantify molecules in simple and complex mixtures. A separation system, such as a liquid chromatograph (LC), gas chromatograph (GC), or capillary electrophoresis (CE) system, may be coupled to the mass spectrometer in a combined system (e.g., LC-MS, GC-MS, or CE-MS system) to separate components in the sample before the components are introduced to the mass spectrometer.

One application of mass spectrometry is the identification, quantification, and structural elucidation of peptides, proteins, and related molecules in complex biological samples. In some such experiments, often referred to as tandem mass spectrometry (MS/MS or MS2) or multi-stage mass spectrometry (MSn where n is 2 or more), certain ions are fragmented in a controlled manner to yield product ions. A mass analysis (referred to as an MS/MS, MS2, or MSn analysis) is then performed on the product ions to generate mass spectra of the product ions. The mass spectra of the product ions provide information that may be used to confirm identification, determine quantity, and/or derive structural details regarding analytes of interest.

Various techniques may be used to acquire mass spectra using tandem mass spectrometry and MSn mass spectrometry. One commonly used technique is data-dependent acquisition (DDA), which uses data acquired in one mass analysis to select, based on predetermined criteria, one or more ion species or a narrow m/z range for mass isolation and fragmentation. For example, the mass spectrometer may perform a full MS survey scan of precursor ions over a wide precursor m/z range and then select one or more precursor ion species from the resulting spectra for subsequent MS/MS or MSn analysis. The criteria for selection of precursor ion species may include intensity, charge state, m/z, inclusion/exclusion lists, or isotopic patterns. The main disadvantage of the DDA technique is the inherently random nature of the results. When technical replicates of the same sample or comparative analysis on other samples is performed, some analytes may be measured in one experiment but not in others. This frustrates attempts to perform reproducible analyses and is known as the "missing value problem".

In contrast to DDA, data-independent acquisition (DIA) is a technique in which all precursor ion species within a wide precursor m/z range (e.g., 500 - 900 m/z) are isolated and fragmented via a sequentially advancing isolation window of a fixed m/z width (e.g., 20 m/z) to generate product ions. An MS2 or MSn analysis is then performed on the product ions in a methodical and unbiased manner. The acquisition of the set of spectra spanning the full precursor m/z range constitutes one cycle, which is repeated to generate MS2 or MSn mass spectra of the product ions. The cycle time, or time required to acquire the spectra in a cycle, is typically set such that at least a certain number of cycles will be executed per chromatography peak width, such that area of the peaks may be properly integrated. In the DIA technique, isolation and fragmentation of one or more precursor ion species is not dependent on data acquired in a survey mass analysis, as in DDA, and is much more suitable for comparing results across different samples than DDA.

In contrast to DDA and DIA, targeted mass spectrometry performs analysis of a fixed list of analytes. Targeted mass spectrometry comes in many forms, such as selected reaction monitoring (SRM), multiple reaction monitoring (MRM), and parallel reaction monitoring (PRM). Generally, the introduction of the sample to the mass spectrometer is performed using a separation system, and to increase throughput the operator schedules analysis of each compound only during a narrow period of time around the characteristic elution times of each analyte of interest. Targeted mass spectrometry is advantageous because of the high data quality that can be produced when the instrument is dedicated to the analysis of a smaller group of analytes of interest, each with a narrow or even customized precursor isolation window. Targeted mass spectrometry produces results with low limits of detection and high dynamic range. However, the throughput of the targeted mass spectrometry analysis is more limited than in techniques like DIA, where the isolation window is usually deliberately made to be broad enough to deliberately multiplex multiple precursor ions.

The DIA technique is well-regarded because it combines the global nature of the older DDA technique with the reproducible and quantitative nature of targeted mass spectrometry techniques. However, due to limitations in instrument speed and sensitivity, there is tension among the isolation width, the precursor m/z range, and the cycle time. Generally, wider isolation widths enable a wider precursor m/z range and thus analysis of a greater number of precursor ion species but produce lower quality data because a wide isolation window may result in co-isolation and co-fragmentation of neighboring analytes, resulting in unidentifiable or low scoring spectra. On the other hand, smaller isolation widths produce better quality data with greater sensitivity at the expense of fewer precursor ion species that may be analyzed due to the narrower precursor m/z range. For example, at the extreme of very narrow isolation widths, the data have the highest quality in terms of sensitivity and selectivity, but the smallest range of precursor ion species are analyzed. Conversely, at the extreme of wide isolation widths, a larger range of precursor ion species may be analyzed but the quality of the resulting data is compromised because the dynamic range of quantitation may suffer and the spectra may have mixed signals produced by many different analytes. Thus, the spectra are difficult to interpret.

### SUMMARY

The following description presents a simplified summary of one or more aspects of the methods and systems described herein to provide a basic understanding of such aspects. This summary is not an extensive overview of all contemplated aspects and is intended to neither identify key or critical elements of all aspects nor delineate the scope of any or all aspects. Its sole purpose is to present some concepts of one or more aspects of the methods and systems described herein in a simplified form as a prelude to the more detailed description that is presented below.

In some illustrative embodiments, a system for performing dynamic data independent acquisition (DIA) comprises a memory storing instructions and a processor communicatively coupled to the memory and configured to execute the instructions to: direct a mass spectrometer to acquire, based on an acquisition schedule that schedules a plurality of acquisition cycles, MS2 spectra of product ions derived from analytes included in a sample as the analytes elute from a separation system, wherein the acquisition schedule specifies, for each acquisition cycle included in the plurality of acquisition cycles, a dynamic precursor m/z range based on an expected elution time of the analytes; and the product ions are produced from precursor ions isolated using an isolation window successively positioned throughout the dynamic precursor m/z range during each acquisition cycle; detect an elution time shift in the elution time of the analytes as the analytes elute from the separation system; and adjust the acquisition schedule based on the detected elution time shift.

In some illustrative embodiments, a non-transitory computer-readable medium storing instructions that, when executed, direct at least one processor of a computing device for mass spectrometry to direct a mass spectrometer to acquire, based on an acquisition schedule that schedules a plurality of acquisition cycles, MS2 spectra of product ions derived from analytes included in a sample as the analytes elute from a separation system, wherein the acquisition schedule specifies, for each acquisition cycle included in the plurality of acquisition cycles, a dynamic precursor m/z range based on an expected elution time of the analytes; and the product ions are produced from precursor ions isolated using an isolation window successively positioned throughout the dynamic precursor m/z range during each acquisition cycle; detect an elution time shift in the elution time of the analytes as the analytes elute from the separation system; and adjust the acquisition schedule based on the detected elution time shift.

In some illustrative embodiments, a method of performing dynamic data independent acquisition (DIA) comprises directing a mass spectrometer to acquire, based on an acquisition schedule that schedules a plurality of acquisition cycles, MS2 spectra of product ions derived from analytes included in a sample as the analytes elute from a separation system, wherein the acquisition schedule specifies, for each acquisition cycle included in the plurality of acquisition cycles, a dynamic precursor m/z range based on an expected elution time of the analytes and the product ions are produced from precursor ions isolated using an isolation window successively positioned throughout the dynamic precursor m/z range during each acquisition cycle; detecting an elution time shift in the elution time of the analytes as the analytes elute from the separation system; and adjusting the acquisition schedule based on the detected elution time shift.

In some illustrative embodiments, a method for performing dynamic data independent acquisition (DIA) comprises obtaining an analyte density matrix associated with a sample, wherein the analyte density matrix represents an expected quantity of analytes included in the sample as a function of m/z and an expected elution time of the analytes from a separation system; and generating, based on the analyte density matrix, an acquisition schedule that schedules a plurality of acquisition cycles for acquisition of DIA MS2 spectra and that specifies, for each acquisition cycle included in the plurality of acquisition cycles, a dynamic precursor m/z range based on the expected elution time of the analytes from the separation system.

### BRIEF DESCRIPTION OF THE DRAWINGS

The accompanying drawings illustrate various embodiments and are a part of the specification. The illustrated embodiments are merely examples and do not limit the scope of the disclosure. Throughout the drawings, identical or similar reference numbers designate identical or similar elements.
FIG. 1 shows a functional diagram of an illustrative LC-MS system.
FIG. 2 shows a functional diagram of an illustrative implementation of mass spectrometer that may be included in the LC-MS system of FIG 1.
FIG. 3 shows a functional diagram of an illustrative mass spectrometry control system that may be used to perform a dynamic DIA experiment.
FIG. 4 shows an illustrative method of performing a dynamic DIA experiment.
FIG. 5 shows a graphical representation of an illustrative analyte density matrix.
FIG. 6 shows a functional diagram of an illustrative scheme for generating the analyte density matrix of FIG. 5 using a gas-phase fractionation technique to characterize the sample.
FIG. 7 shows a static precursor m/z range and a dynamic acquisition schedule in relation to the analyte density matrix of FIG. 5.
FIG. 8A shows a chart illustrating the distribution of the maximum isolation width possible for the analytes represented in the analyte density matrix of FIG. 5.
FIG. 8B shows a chart that indicates the number of analytes that can be quantified using a given isolation width, the number of analytes that can be analyzed with scheduled MS2 acquisitions, and the number of analytes that are both quantifiable and can be analyzed with scheduled MS2 acquisitions.
FIG. 9 shows an illustrative scheme for acquiring MS2 spectra by dynamic DIA based on the acquisition schedule of FIG. 7.
FIG. 10 shows an illustrative method of detecting elution time shift using alignment spectra acquired during the dynamic DIA experiment.
FIG. 11 shows an illustrative scheme for acquiring alignment spectra during a dynamic DIA experiment.
FIG. 12 shows an illustrative technique for detecting elution time shift by cross-correlating a set of alignment spectra with a set of reference spectra.
FIG. 13 shows an illustrative computing device.

### DETAILED DESCRIPTION

Systems, apparatuses, and methods of performing dynamic data independent acquisition (DIA) are described herein. For example, a system for performing dynamic data independent acquisition is configured to direct a mass spectrometer to acquire, based on an acquisition schedule that schedules a plurality of acquisition cycles, MS2 spectra of product ions derived from analytes included in a sample as the analytes elute from a separation system. The acquisition schedule specifies, for each acquisition cycle included in the plurality of acquisition cycles, a dynamic precursor m/z range based on an expected elution time of the analytes. The product ions are produced from precursor ions isolated using an isolation window successively positioned throughout the dynamic precursor m/z range during each acquisition cycle. The system is further configured to detect an elution time shift in the elution time of the analytes as the analytes elute from the separation system and adjust the acquisition schedule based on the detected elution time shift.

The systems, apparatuses, and methods described for performing dynamic (DIA) maintain a high degree of analyte coverage over time, even as analyte elution time shifts. With dynamic DIA, the dynamic precursor m/z range used at any point in time allows analysis of the highest density precursor m/z region. Dynamic DIA also enables narrower isolation widths that yield higher quality data, although at a sometimes minor cost on the analyte coverage of the analysis. However, the analyte coverage loss is relatively small given that the dynamic precursor m/z range covers the highest density precursor m/z region. As an alternative or in concert with using narrower isolation widths, dynamic DIA allows for the acquisitions to accumulate ions for a longer time, which allows for characterization of lower abundance analytes, and may increase the quality of the analysis.

Various embodiments will now be described in more detail with reference to the figures. The systems and methods described herein may provide one or more of the benefits mentioned above and/or various additional and/or alternative benefits that will be made apparent herein.

In some implementations, the methods and systems for performing ion population regulation may be used in conjunction with a combined separation-mass spectrometry system, such as an LC-MS system. As such, an LC-MS system will now be described. The described LC-MS system is illustrative and not limiting. The methods and systems described herein may operate as part of or in conjunction with the LC-MS system described herein and/or with any other suitable separation-mass spectrometry system, including a high-performance liquid chromatography-mass spectrometry (HPLC-MS) system, a gas chromatography-mass spectrometry (GC-MS) system, or a capillary electrophoresis-mass spectrometry (CE-MS) system. The methods and systems described herein may also operate in conjunction with any other continuous flow sample source, such as a flow-injection mass spectrometry system (FI-MS) in which analytes are injected into a mobile phase (without separation in a column) and enter the mass spectrometer with time-dependent variations in intensity (e.g., Gaussian-like peaks).

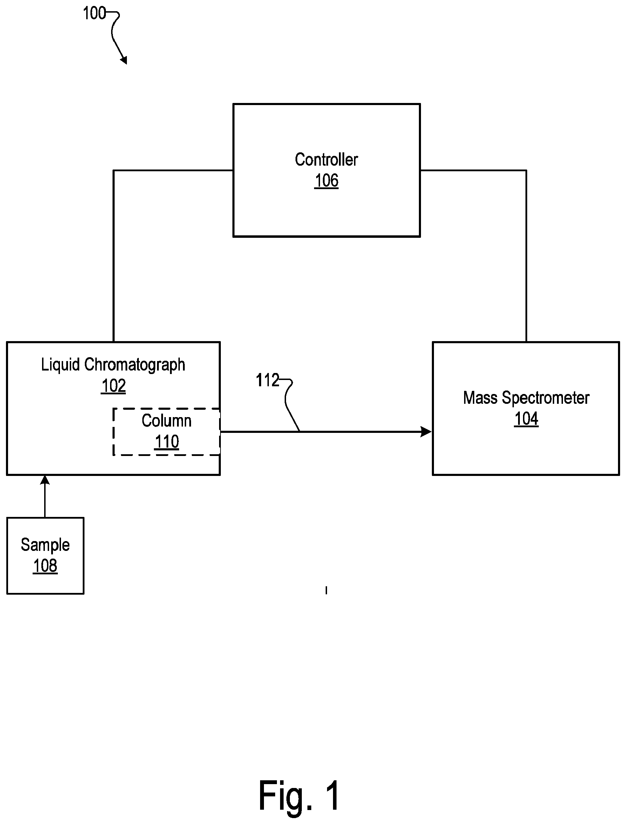

FIG. 1 shows a functional diagram of an illustrative LC-MS system 100. LC-MS system 100 includes a liquid chromatograph 102, a mass spectrometer 104, and a controller 106. Liquid chromatograph 102 is configured to separate, over time, components (e.g., analytes) within a sample 108 that is injected into liquid chromatograph 102. Sample 108 may include, for example, chemical components (e.g., molecules, ions, etc.) and/or biological components (e.g., metabolites, proteins, peptides, lipids, etc.) for detection and analysis by LC-MS system 100. Liquid chromatograph 102 may be implemented by any liquid chromatograph as may suit a particular implementation. In liquid chromatograph 102, sample 108 may be injected into a mobile phase (e.g., a solvent), which carries sample 108 through a column 110 containing a stationary phase (e.g., an adsorbent packing material). As the mobile phase passes through column 110, components within sample 108 elute from column 110 at different times based on, for example, their size, their affinity to the stationary phase, their polarity, and/or their hydrophobicity.

A detector (e.g., an ion detector component of mass spectrometer 104, an ion-electron converter and electron multiplier, etc.) may measure the relative intensity of a signal modulated by each separated component in eluate 112 from column 110. Data generated by the detector may be represented as a chromatogram, which plots retention time on the x-axis and a signal representative of the relative intensity on the y-axis. The retention time of a component is generally measured as the period of time between injection of sample 108 into the mobile phase and the relative intensity peak maximum after chromatographic separation. In some examples, the relative intensity may be correlated to or representative of relative abundance of the separated components. Data generated by liquid chromatograph 102 may be output to controller 106.

In some cases, particularly in analyses of complex mixtures, multiple different components in sample 108 co-elute from column 110 at approximately the same time, and thus may have the same or similar retention times. As a result, determination of the relative intensity of the individual components within sample 108 requires further separation of signals attributable to the individual components. To this end, liquid chromatograph 102 directs components included in eluate 112 to mass spectrometer 104 for identification and/or quantification of one or more of the components.

Mass spectrometer 104 is configured to produce ions from the components received from liquid chromatograph 102 and sort or separate the produced ions based on m/z of the ions. A detector in mass spectrometer 104 measures the intensity of the signal produced by the ions. As used herein, "intensity" or "signal intensity" refers to the response of the detector and may represent absolute abundance, relative abundance, ion count, intensity, relative intensity, ion current, or any other suitable measure of ion detection. Data generated by the detector may be represented by mass spectra, which plot the intensity of the observed signal as a function of m/z of the detected ions. Data acquired by mass spectrometer 104 may be output to controller 106.

Mass spectrometer 104 may be implemented by a tandem mass spectrometer configured to perform tandem mass spectrometry (denoted MS/MS or MS2) or a multi-stage mass spectrometer configured to perform multi-stage mass spectrometry (also denoted MSⁿ).

Controller 106 may be communicatively coupled with, and configured to control operations of, LC-MS system 100 (e.g., liquid chromatograph 102 and mass spectrometer 104). Controller 106 may include any suitable hardware (e.g., a processor, circuitry, etc.) and/or software configured to control operations of and/or interface with the various components of LC-MS system 100 (e.g., liquid chromatograph 102 or mass spectrometer 104).

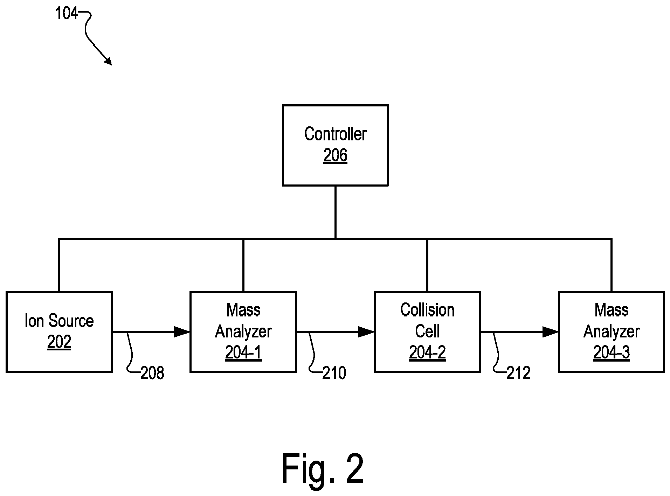

FIG. 2 shows a functional diagram of an illustrative implementation of mass spectrometer 104. As shown, mass spectrometer 104 is tandem-in-space (e.g., has multiple mass analyzers) and has two stages for performing MS/MS. However, mass spectrometer 104 is not limited to this configuration but may have any other suitable configuration. For example, mass spectrometer 104 may be tandem-in-time. Additionally or alternatively, mass spectrometer 104 may be a multi-stage mass spectrometer and may have any suitable number of stages (e.g., three or more) for performing multi-stage tandem mass spectrometry (e.g., MS/MS/MS). As used herein, tandem mass spectrometry refers to MS/MS as well as MSn mass spectrometry where n is greater than two.

As shown, mass spectrometer 104 includes an ion source 202, a first mass analyzer 204-1, a collision cell 204-2, a second mass analyzer 204-3, and a controller 206. Mass spectrometer 104 may further include any additional or alternative components not shown as may suit a particular implementation (e.g., ion optics, filters, ion stores, an autosampler, a detector, etc.).

Ion source 202 is configured to produce a stream 208 of ions from the components and deliver the ions to first mass analyzer 204-1. Ion source 202 may use any suitable ionization technique, including without limitation electron ionization, chemical ionization, matrix assisted laser desorption/ionization, electrospray ionization, atmospheric pressure chemical ionization, atmospheric pressure photoionization, inductively coupled plasma, and the like. Ion source 202 may include various components for producing ions from components included in sample 108 and delivering the ions to first mass analyzer 204-1.

First mass analyzer 204-1 is configured to receive ion stream 208, isolate precursor ions of a selected m/z range and deliver a beam 210 of precursor ions to collision cell 204-2. Collision cell 204-2 is configured to receive beam 210 of precursor ions and produce product ions (e.g., fragment ions) via controlled dissociation processes. Collision cell 204-2 is further configured to direct a beam 212 of product ions to second mass analyzer 204-3. Second mass analyzer 204-3 is configured to filter and/or perform a mass analysis of the product ions.

Mass analyzers 204-1 and 204-3 are configured to isolate or separate ions according to m/z of each of the ions. Mass analyzers 204-1 and 204-3 may be implemented by any suitable mass analyzer, such as a quadrupole mass filter, an ion trap (e.g., a three-dimensional quadrupole ion trap, a cylindrical ion trap, a linear quadrupole ion trap, a toroidal ion trap, etc.), a time-of-flight (TOF) mass analyzer, an electrostatic trap mass analyzer (e.g. an orbital electrostatic trap such as an Orbitrap mass analyzer, a Kingdon trap, etc.), a Fourier transform ion cyclotron resonance (FT-ICR) mass analyzer, and the like. Mass analyzers 204-1 and 204-3 need not be implemented by the same type of mass analyzer.

Collision cell 204-2 may be implemented by any suitable collision cell. As used herein, "collision cell" may encompass any structure or device configured to produce product ions via controlled dissociation processes and is not limited to devices employed for collisionally-activated dissociation. For example, collision cell 204-2 may be configured to fragment precursor ions using collision induced dissociation, electron transfer dissociation, electron capture dissociation, photo induced dissociation, surface induced dissociation, ion/molecule reactions, and the like.

An ion detector (not shown) is configured to detect ions at each of a variety of different m/z and responsively generate an electrical signal representative of ion intensity. The electrical signal is transmitted to controller 206 for processing, such as to construct a mass spectrum of the sample. For example, mass analyzer 204-3 may emit an emission beam of separated ions to the ion detector, which is configured to detect the ions in the emission beam and generate or provide data that can be used by controller 206 to construct a mass spectrum of the sample. The ion detector may be implemented by any suitable detection device, including without limitation an electron multiplier, a Faraday cup, and the like.

Controller 206 may be communicatively coupled with, and configured to control operations of, mass spectrometer 104. For example, controller 206 may be configured to control operation of various hardware components included in ion source 202 and/or mass analyzers 204-1 and 204-3. To illustrate, controller 206 may be configured to control an accumulation time of ion source 202 and/or mass analyzers 204, control an oscillatory voltage power supply and/or a DC power supply to supply an RF voltage and/or a DC voltage to mass analyzers 204, adjust values of the RF voltage and DC voltage to select an effective m/z (including a mass tolerance window) for analysis, and adjust the sensitivity of the ion detector (e.g., by adjusting the detector gain).

Controller 206 may also include and/or provide a user interface configured to enable interaction between a user of mass spectrometer 104 and controller 206. The user may interact with controller 206 via the user interface by tactile, visual, auditory, and/or other sensory type communication. For example, the user interface may include a display device (e.g., liquid crystal display (LCD) display screen, a touch screen, etc.) for displaying information (e.g., mass spectra, notifications, etc.) to the user. The user interface may also include an input device (e.g., a keyboard, a mouse, a touchscreen device, etc.) that allows the user to provide input to controller 206. In other examples the display device and/or input device may be separate from, but communicatively coupled to, controller 206. For instance, the display device and the input device may be included in a computer (e.g., a desktop computer, a laptop computer, etc.) communicatively connected to controller 206 by way of a wired connection (e.g., by one or more cables) and/or a wireless connection.

Controller 206 may include any suitable hardware (e.g., a processor, circuitry, etc.) and/or software as may serve a particular implementation. While FIG. 2 shows that controller 206 is included in mass spectrometer 104, controller 206 may alternatively be implemented in whole or in part separately from mass spectrometer 104, such as by a computing device communicatively coupled to mass spectrometer 104 by way of a wired connection (e.g., a cable) and/or a network (e.g., a local area network, a wireless network (e.g., Wi-Fi), a wide area network, the Internet, a cellular data network, etc.). In some examples, controller 206 may be implemented in whole or in part by controller 106.

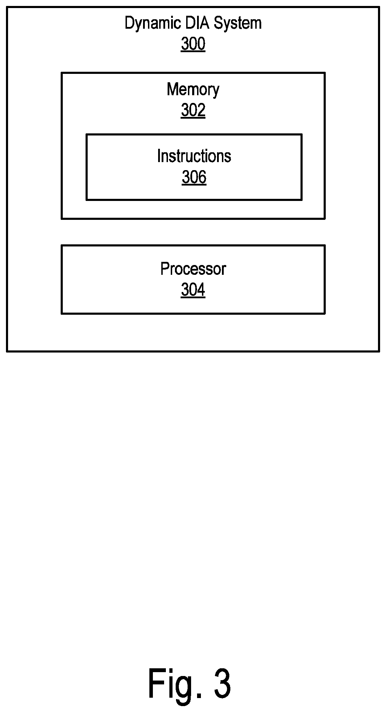

LC-MS system 100 may be used to perform a dynamic DIA experiment to analyze a sample in conjunction with a mass spectrometry control system. The mass spectrometry control system may control and/or perform one or more operations associated with performing a DIA experiment. FIG. 3 shows a functional diagram of an illustrative dynamic DIA system 300 ("system 300"). System 300 may be implemented entirely or in part by LC-MS system 100 (e.g., by controller 106 and/or controller 206). Alternatively, system 300 may be implemented separately from LC-MS system 100 (e.g., a remote computing system or server separate from but communicatively coupled to controller 106 and/or controller 206).

System 300 may include, without limitation, a memory 302 and a processor 304 selectively and communicatively coupled to one another. Memory 302 and processor 304 may each include or be implemented by hardware and/or software components (e.g., processors, memories, communication interfaces, instructions stored in memory for execution by the processors, etc.). In some examples, memory 302 and processor 304 may be distributed between multiple devices and/or multiple locations as may serve a particular implementation.

Memory 302 may maintain (e.g., store) executable data used by processor 304 to perform any of the operations described herein. For example, memory 302 may store instructions 306 that may be executed by processor 304 to perform any of the operations described herein. Instructions 306 may be implemented by any suitable application, software, code, and/or other executable data instance.

Memory 302 may also maintain any data acquired, received, generated, managed, used, and/or transmitted by processor 304. For example, memory 302 may maintain LC-MS data (e.g., acquired chromatogram data and/or mass spectra data), an analyte density matrix and/or a set of reference spectra, which are described in detail below.

Processor 304 may be configured to perform (e.g., execute instructions 306 stored in memory 302 to perform) various processing operations described herein. It will be recognized that the operations and examples described herein are merely illustrative of the many different types of operations that may be performed by processor 304. In the description herein, any references to operations performed by system 300 may be understood to be performed by processor 304 of system 300. Furthermore, in the description herein, any operations performed by system 300 may be understood to include system 300 directing or instructing another system or device to perform the operations.

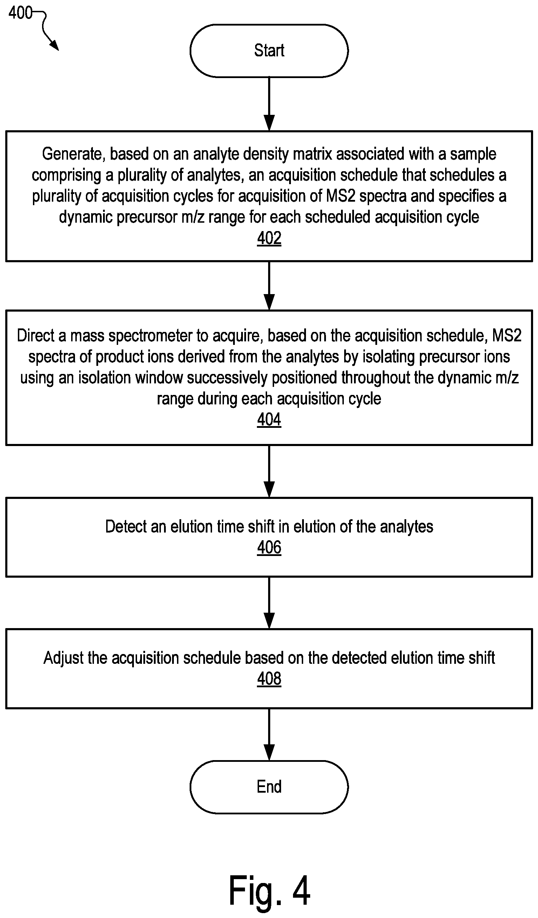

FIG. 4 shows an illustrative method 400 of performing a dynamic DIA experiment. While FIG. 4 shows illustrative operations according to one embodiment, other embodiments may omit, add to, reorder, and/or modify any of the operations shown in FIG. 4. One or more of the operations shown in FIG. 4 may be performed by LC-MS system 100 and/or system 300, any components included therein, and/or any implementations thereof (e.g., mass spectrometer 104, one or more components of mass spectrometer 104, and/or a remote computing system separate from but communicatively coupled to mass spectrometer 104).

At operation 402, system 300 generates, based on an analyte density matrix associated with a sample (e.g., sample 108), an acquisition schedule for acquisition, by DIA, of MS2 spectra of ions derived from analytes included in the sample as the analytes elute from a separation system (e.g., liquid chromatograph 102). The analyte density matrix represents an expected quantity of quantifiable analytes as a function of m/z of precursor ions derived from the analytes and elution time of the analytes. For chromatographic separation applications, elution time refers to retention time, which is generally measured as the period of time between injection of the sample into the mobile phase and the relative intensity peak maximum after chromatographic separation. For capillary electrophoresis applications, in which analytes are not retained but instead continuously migrate, elution time refers to migration time. Migration time is generally measured as the period of time taken for an analyte to migrate from the beginning of the capillary to a detection point.

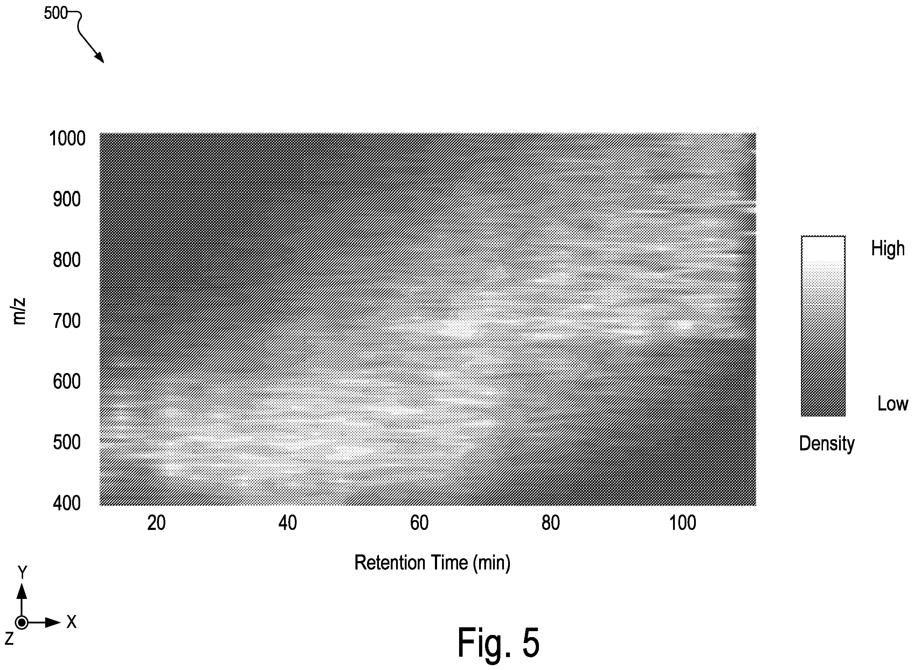

FIG. 5 shows a graphical representation of an illustrative analyte density matrix 500 for a biological sample having approximately 20,000 quantifiable peptides. In analyte density matrix 500, the x axis indicates the elution time (in minutes), the y axis indicates m/z of precursor ions of the peptides, and the z axis indicates quantity of the precursor ions. Thus, analyte density matrix 500 represents the quantity of peptides at different values of m/z over elution time as the peptides elute from a separation system. The lighter color represents a higher density of peptides and the darker color represents a lower density of peptides. As can be seen, the density of eluting peptides varies as a function of m/z and elution time of the peptides.

Referring again to FIG. 4, the analyte density matrix is generated prior to performing the dynamic DIA experiment (e.g., prior to injection of the sample into the separation system for dynamic DIA MS2 acquisition and/or prior to initiating method 400). The analyte density matrix may be generated in any suitable way.

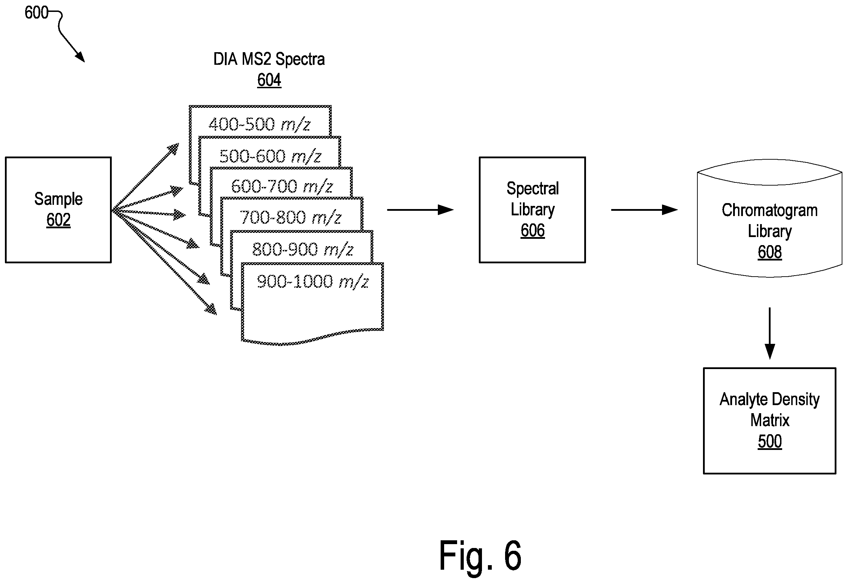

In some examples, the analyte density matrix is generated experimentally by a characterization analysis. FIG. 6 shows a functional diagram of an illustrative scheme 600 for generating analyte density matrix 500 using a gas-phase fractionation technique to characterize the sample. As shown, multiple injections of a sample 602 (e.g., sample 108) are analyzed by a combined separation-mass spectrometry system (e.g., LC-MS system 100). System 300 directs a mass spectrometer to acquire MS2 characterization spectra 604 for each injection by analyzing the product ions using a narrow isolation window successively positioned throughout a narrow subset of a characterization m/z range of interest as the analytes elute from the separation system. As shown in FIG. 6, sample 602 is injected six different times, with each injection spanning 100 m/z of the characterization m/z range of interest spanning from 400 to 1000 m/z. In some examples, the isolation width of the isolation window is greater than 0 m/z but less than or equal to about 4 m/z. In other examples, the isolation width of the isolation window is greater than 0 m/z but less than or equal to about 2 m/z. In yet further examples, the isolation width is greater than 0 m/z but less than or equal to about 1 m/z. By using a narrow subset of the characterization m/z range of interest and a narrow isolation width, the sample can be analyzed with high sensitivity and precision. The characterization m/z range of interest, the subset of the characterization m/z range of interest, and the number of injections need not be as shown in FIG. 6 but may be modified as may suit a particular implementation.

System 300 compares the MS2 characterization spectra 604 against a spectral library 606 to identify spectral matches and build a chromatogram library 608. Chromatogram library 608 records elution time, precursor m/z, peptide fragmentation patterns, and known interferences that identify each analyte within sample 602. In this way, the quantity of eluting peptides may be determined. System 300 may use chromatogram library 608 to generate analyte density matrix 500. The analytes detected in MS2 characterization spectra 604 may be filtered, based on the number of transitions, time correlation, experimental relevance (e.g., target analytes of interest), and/or total area, for characteristics that enable high quality analysis. For instance, quantifiable peptides may include peptides that have at least 4 transitions with a time correlation greater than or equal to 0.9 and a total area greater than 1000. However, other parameters and/or parameter values may be selected to determine the population of peptides that are quantifiable.

Alternatively to generating the analyte density matrix experimentally as in scheme 600, the analyte density matrix may be generated *in silico,* such as by using a deep learning model (e.g., Prosit, developed by The ProteomeTools Project) that generates predicted MS2 characterization spectra and elution times. The predicted MS2 characterization spectra may be compared against a spectral library to identify spectral matches and build a chromatogram library, which, as described above, may be used to generate the analyte density matrix.

As mentioned, system 300 uses the analyte density matrix to generate an acquisition schedule for a dynamic DIA experiment. The acquisition schedule schedules a plurality of acquisition cycles for acquisition of MS2 spectra and specifies, for each acquisition cycle, a dynamic precursor m/z range, within a characterization m/z range of interest, based on the expected elution time of analytes included in the sample. In some examples, as will be described below, the acquisition schedule also schedules acquisition of alignment spectra that may be used to perform elution time adjustment of the acquisition schedule to account for elution time drift of the analytes.

The characterization m/z range of interest is the wide precursor m/z range covered by the analyte density matrix and is the precursor m/z range analyzed in the characterization analysis of the sample. In analyte density matrix 500, the characterization m/z range of interest spans 600 m/z and extends from 400 m/z to 1000 m/z. The dynamic precursor m/z range is a narrower m/z range within the characterization m/z range of interest. Ions having an m/z within the dynamic precursor m/z range are isolated by a first mass analyzer (e.g., mass analyzer 204-1) and delivered to a collision cell (e.g., collision cell 204-2) for fragmentation into product ions. The product ions are delivered to a second mass analyzer (e.g., mass analyzer 204-3) and analyzed to produce MS2 mass spectra.

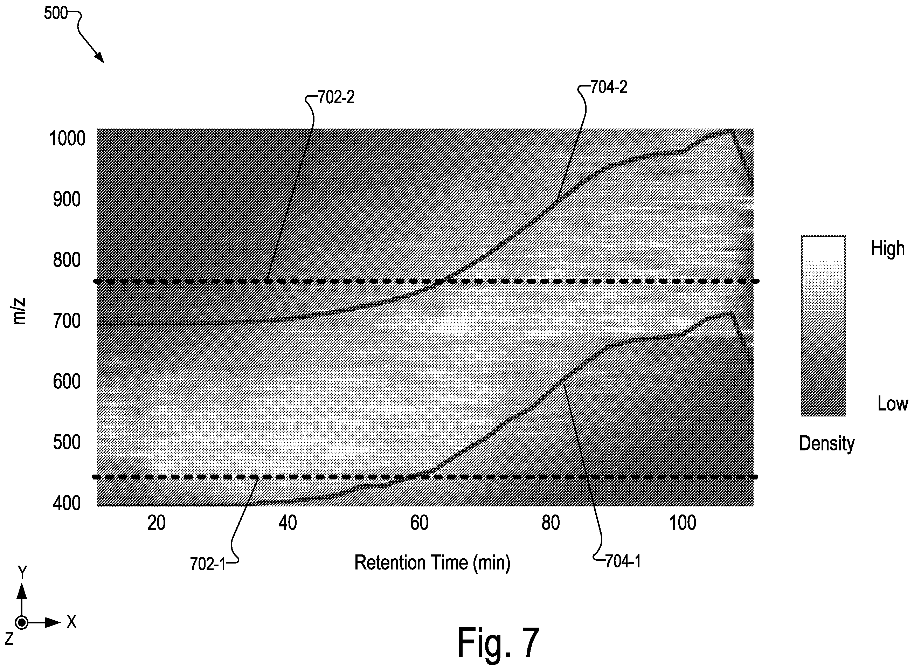

Conventional methods of performing DIA use a static precursor m/z range. For instance, FIG. 7 shows analyte density matrix 500 with a static precursor m/z range having lower and upper bounds indicated on analyte density matrix 500 by dashed lines 702-1 and 702-2, respectively. As shown, the static precursor m/z range spans 300 m/z from 450 m/z to 750 m/z over the entire course of the DIA experiment. However, as shown in analyte density matrix 500, the regions of analyte density matrix 500 with the highest peptide density varies over time. For example, at an elution time of 20 minutes, the highest density of peptides occurs between about 400 m/z and 700 m/z. At an elution time of 60 minutes, the highest density of peptides occurs between about 400 m/z and 900 m/z. At an elution time of 80 minutes, the highest density of peptides occurs between about 600 m/z and 1000 m/z. As a result, the static precursor m/z range analyzes significant regions of analyte density matrix 500 with little to no detectable ions (e.g., the upper left corner and the lower right corner within the static precursor m/z range) while significant m/z regions with high peptide density (e.g., the top of analyte density matrix 500 after 60 minutes) are excluded from analysis. As a result, a relatively small percentage (e.g., 60%) of quantifiable peptides might be analyzed.

In order to optimize the number of peptides that are analyzed over the course of the experiment, system 300 generates an acquisition schedule that schedules a plurality of acquisition cycles for acquisition of MS2 spectra and specifies, for each acquisition cycle, a dynamic precursor m/z range based on the expected elution time of the analytes. As used herein, a dynamic precursor m/z range has upper and lower bounds that vary as a function of elution time. Accordingly, the acquisition schedule specifies upper and lower bounds of the dynamic precursor m/z range as well as a position, along the m/z domain of the analyte density matrix, of the dynamic precursor m/z range.

FIG. 7 shows a graphical representation of an illustrative acquisition schedule for dynamic DIA and that may be generated based on analyte density matrix 500. As shown, solid lines 704-1 and 704-2 on analyte density matrix 500 indicate a lower bound and an upper bound, respectively, of a dynamic precursor m/z range. The dynamic precursor m/z range is centered along the m/z domain over time so as to optimize the number of peptides that are analyzed by DIA MS2.

As used herein, "optimize" and its variants means to seek an improved or optimum solution among a set of possible solutions, although the best solution may not necessarily be obtained, such as when an optimization process is terminated prior to finding the best solution, when multiple solutions exist that satisfy predefined criteria, when a solution satisfies minimum criteria, or when a selected optimization technique is unable to converge on the best solution. Similarly, as used herein an "optimum" parameter (e.g., a "maximum" or "minimum" value of a parameter) means the solution obtained as the result of performing an optimization process, and thus may not necessarily be the absolute extreme value of the parameter (e.g., the absolute maximum or minimum), but still adjusts the parameter and results in an improvement.

The span of the dynamic precursor m/z range that can be analyzed in an acquisition cycle generally depends on the isolation width, instrument speed, and cycle time. The instrument speed is the speed at which the instrument can perform one acquisition over the isolation width. The cycle time for an acquisition cycle is the amount of time the instrument takes to acquire a set of mass spectra over the entire dynamic precursor m/z range. For example, with 2 m/z isolation width, 75 Hz instrument speed, and 2 seconds cycle time, system 300 may distribute 150 acquisitions per acquisition cycle covering a precursor m/z range of 300 m/z along the m/z domain to best capture the precursor ions of analytes expected to elute at any given time.

In some examples, system 300 determines the isolation width for the acquisition cycles based on a chromatogram library, such as the chromatogram library used to generate the analyte density matrix (e.g., chromatogram library 608). The chromatogram library may indicate a distribution of the maximum isolation width possible for any analyte while maintaining a minimum number of quantifiable transitions (e.g., 4, 5, etc.) for the analyte.

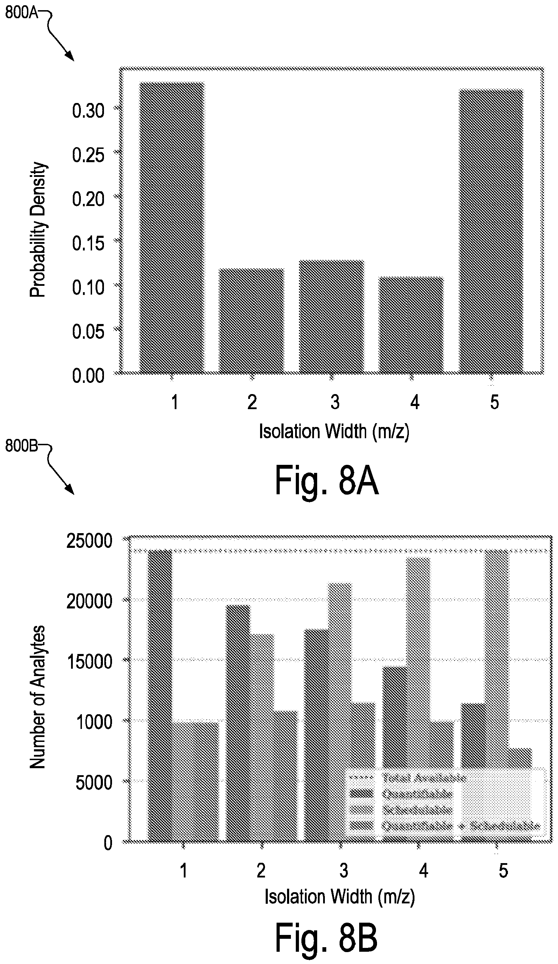

FIG. 8A shows a chart 800A illustrating the distribution of the maximum possible isolation width for the analytes represented in analyte density matrix 500. In this context, the maximum possible isolation width refers to the maximum width for which a precursor's transitions may be analyzed with a minimum acceptable quality. Illustrative examples of determining the maximum possible isolation width are described in International Publication No. WO2022/106948A1 , which is incorporated herein by reference in its entirety. In the example of FIG. 8A, chart 800A is based on chromatogram library 608 generated with an isolation width of 1 m/z. As shown in chart 800A, of the 20,000 quantifiable peptides represented by analyte density matrix 500, 33% have a maximum isolation width of 1 m/z, 11% have a maximum isolation width of 2 m/z, 12% have a maximum isolation width of 3 m/z, 11% have a maximum isolation width of 4 m/z, and 33% have a maximum isolation width of 5 m/z. System 300 may use this isolation width information when generating the acquisition schedule to determine a total number of analytes that are both quantifiable and can be schedulable by scheduled DIA using a selected isolation width. For example, FIG. 8B shows a chart 800B that indicates the number of analytes that can be quantified using a given isolation width (e.g., that maintain a minimum number of transitions with the given isolation width), the number of analytes that can be analyzed with scheduled MS2 acquisitions, and the number of analytes that are both quantifiable and can be analyzed with scheduled MS2 acquisitions. FIG. 8B illustrates the competing requirements of quality and coverage. In the example of FIG. 8B, the 3 m/z isolation width provides an optimum number peptides that can be analyzed with scheduled MS2 acquisitions. Accordingly, system 300 sets the isolation width at 3 m/z. The acquisition schedule specifies the isolation width for the acquisition cycles.

In some examples, the isolation width of the isolation window is greater than 0 m/z but less than or equal to about 4 m/z. In other examples, the isolation width of the isolation window is greater than 0 m/z but less than or equal to about 2 m/z. In yet further examples, the isolation width is greater than 0 m/z but less than or equal to equal about 1 m/z. In some examples, the isolation width used for acquisition cycles varies over time. For example, the acquisition schedule may use larger (or smaller) isolation widths for acquisition cycles that occur later in the dynamic DIA experiment. These time-varying isolation widths can be determined in the same way as described above with reference to FIGS. 8A and 8B, by separating the analytes into time bins for each isolation width determination.

In some examples, the acquisition cycle time is determined based on the width of the elution peaks of the analytes as the analytes are eluting. Accordingly, system 300 may set the acquisition cycle time based on the width of the elution peaks recorded in the chromatogram library generated from the characterization analysis of the sample.

In some examples, the span of the dynamic precursor m/z range remains constant (e.g., is the same at any point in time). For example, as shown in FIG. 7, the span of the dynamic precursor m/z range remains constant over time (e.g., 300 m/z). In other examples, the span of the dynamic precursor m/z range also varies over time, such as when system 300 uses time-varying isolation widths over the course of the dynamic DIA experiment, as described above.

As shown in FIG. 7, the precursor m/z range is continuous from the lower bound to the upper bound. In other examples, the precursor m/z range is noncontinuous at one or more points in time. That is, the precursor m/z range may have multiple non-contiguous spans, each with a different upper and lower bound.

With a dynamic precursor m/z range, a larger number of analytes may be analyzed by DIA than with conventional DIA methods. In the example of FIG. 7, the acquisition schedule with a dynamic precursor m/z range may result in 70% or more of quantifiable peptides being analyzed.

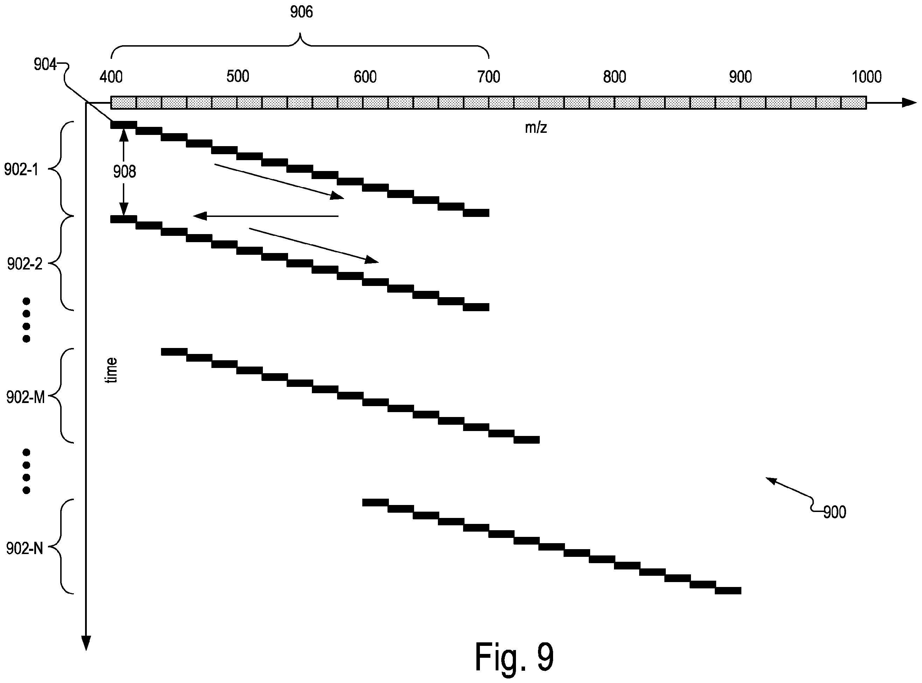

Returning to FIG. 4, at operation 404, system 300 directs a mass spectrometer to acquire, based on the acquisition schedule, MS2 spectra of product ions derived from the analytes included in the sample as the analytes elute. FIG. 9 shows an illustrative scheme 900 for acquiring MS2 spectra by dynamic DIA based on the acquisition schedule. As shown in scheme 900, a plurality of acquisition cycles 902 (e.g., acquisition cycles 902-1, 902-2, 902-M, and 902-N) are scheduled for acquisition of MS2 spectra. A scheduled acquisition cycle 902-1 is performed by isolating precursor ions having an m/z within a small isolation window 904 of a fixed isolation width, fragmenting the isolated precursor ions into product ions, and performing an MS2 analysis of the product ions to acquire an MS2 spectrum. Isolation window 904 is successively positioned throughout a dynamic precursor m/z range 906 over time during acquisition cycle 902-1 to cover the entire dynamic m/z range 906, thereby acquiring a set of MS2 mass spectra that can be used for identification and quantitation of the analytes. The first dynamic precursor m/z range 906 ranges from 400 m/z to 900 m/z. The time taken to acquire the set of MS spectra covering the dynamic precursor m/z range 906 is the cycle time, represented by arrow 908. In the example of FIG. 9, isolation window 904 has a fixed isolation width of 20 m/z and dynamic precursor m/z range 906 spans 300 m/z. Accordingly, the scheduled MS2 acquisition cycle includes 15 acquisitions.

It will be understood that FIG. 9 is merely illustrative, as the isolation width of isolation window 904, the cycle time, the span of dynamic precursor m/z range 906, and/or the order of successive positioning of isolation window 904 may be different as may suit a particular implementation. In some examples, the isolation width is not fixed within acquisition cycle 902-1 but varies based on m/z. For example, the isolation width may be larger (or smaller) at edge regions of dynamic precursor m/z range 906 (e.g., from 400-500 m/z and 900-1000 m/z) than at a center region of dynamic precursor m/z range 906. In the example of FIG. 9, isolation window 904 is successively positioned within dynamic precursor m/z range 906 in a sequential manner (e.g., from lowest to highest m/z). However, isolation window 904 may be successively positioned throughout dynamic precursor m/z range 906 in any other order or pattern (e.g., from highest to lowest m/z or non-sequentially along the m/z domain, such as staggered or alternating), or randomly.

After acquisition cycle 902-1 is completed, another acquisition cycle 902-2 is performed in the same manner, and the process is continuously repeated as the analytes elute from the separation system. As explained above, system 300 directs the mass spectrometer to acquire the MS2 spectra based on the acquisition schedule that specifies the dynamic precursor m/z range for each acquisition cycle as a function of the expected elution time of the analytes. In the example of FIG. 9, the acquisition schedule specifies that the dynamic precursor m/z range for an Mth acquisition cycle 902-M ranges from 440 m/z to 940 m/z, and the dynamic precursor m/z range for an Nth acquisition cycle 902-N ranges from 600 m/z to 900 m/z. Accordingly, the Mth acquisition cycle 902-M begins at 440 m/z and the Nth acquisition cycle 902-N begins at 600 m/z. By performing dynamic DIA with a dynamic precursor m/z range, the balance between the number of analytes that may be analyzed by MS2 and the data quality may be optimized. Since a greater number of analytes can be analyzed using a dynamic precursor m/z, the isolation width can be made narrower to improve sensitivity and data quality.

Analyte elution times may vary from time to time and shift as the chromatographic column ages. This elution time shift (also referred to as retention time shift in chromatographic applications or elution time drift) can cause issues with the reproducibility of a dynamic DIA analysis, because as the elution times shift, analytes on the "edges" of the dynamic precursor m/z range may be missing in some replicates. Thus, the scheduled acquisitions fail to overlap well with the intended analyte coverage.

Returning to FIG. 4, to address the problem of elution time shift, system 300 performs operations 406 and 408 to monitor for elution time shift and, when detected, perform an elution time alignment of the acquisition schedule. At operation 406, system 300 detects, during the dynamic DIA experiment (e.g., during acquisition of MS spectra based on the acquisition schedule), an elution time shift in the elution of the analytes. At operation 408, system 300 adjusts, during the dynamic DIA experiment, the acquisition schedule based on the elution time shift.

System 300 may detect the elution time shift (operation 406) in any suitable way. In some examples, system 300 detects the elution time shift by monitoring for internal standards (e.g., stable isotope-labeled peptide analogs) or elution time markers (e.g., indexed elution time standards) spiked into the sample, monitoring for a known high abundant species, machine learning approaches for chromatographic alignment, and/or any other method, including methods used in data dependent acquisitions and/or targeted acquisitions.

In other examples, system 300 detects the elution time shift by comparing current mass spectra acquired at or near a current time during the dynamic DIA experiment with reference spectra acquired before the dynamic DIA experiment. The current mass spectra may be alignment spectra acquired in in one or more alignment cycles performed during the dynamic DIA experiment, or the current mass spectra may be MS2 spectra acquired in a scheduled acquisition cycle of the dynamic DIA experiment. The reference spectra are acquired prior to the dynamic DIA experiment, such as by performing a reference run of the sample, and represent an expected elution profile of the sample. The current mass spectra and the reference spectra may be raw mass spectra data or may be mass spectra data that has been processed to facilitate the comparison, such as by binning, de-noising, compressing, transforming (e.g., performing a wavelet transform), feature extraction, projecting to a fixed time grid, etc. Based on the comparison, system 300 determines where the current time of the dynamic DIA experiment is in relation to an expected time of the dynamic DIA experiment, as indicated by the reference spectra.

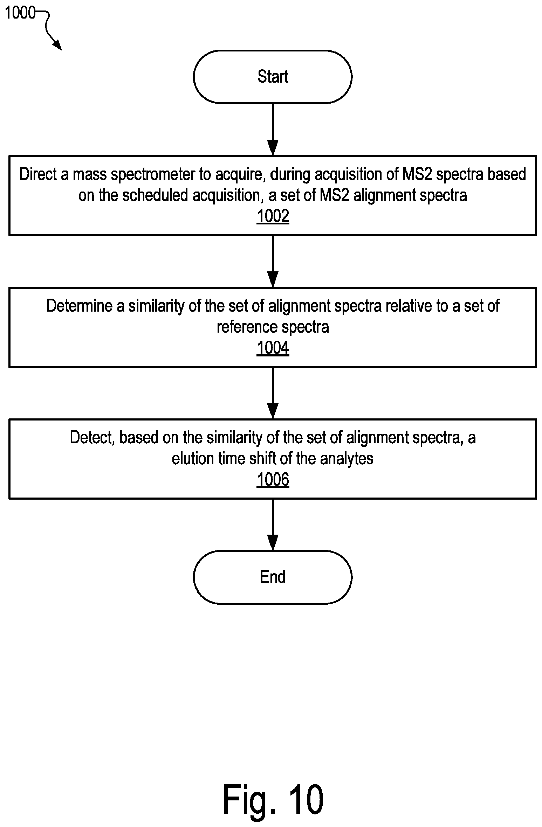

FIG. 10 shows an illustrative method 1000 of detecting elution time shift using alignment spectra acquired during the dynamic DIA experiment. While FIG. 10 shows illustrative operations according to one embodiment, other embodiments may omit, add to, reorder, and/or modify any of the operations shown in FIG. 10.

At operation 1002, system 300 directs a mass spectrometer to acquire, during acquisition of the MS2 spectra based on the acquisition schedule (e.g., between two scheduled acquisition cycles), a set of alignment spectra. The set of alignment spectra includes alignment spectra acquired in one or more (e.g., 2, 3, 5, etc.) alignment cycles by DIA MS2 by isolating precursor ions using an isolation window successively positioned throughout an alignment precursor m/z range during each alignment cycle. In some examples, the alignment precursor m/z range is wider than the dynamic precursor m/z range. In some examples, the alignment precursor m/z range is the characterization m/z range of interest of the analyte density matrix or the precursor m/z range of interest used during the reference run. The isolation window may have a relatively wide isolation width so that the alignment precursor m/z range may be analyzed. In some examples, the isolation window has an isolation width equal to or greater than about 10 m/z. In further examples, the isolation window has an isolation width equal to or greater than about 15 m/z. In yet further examples, the isolation window has an isolation width equal to or greater than about 20 m/z. In some examples, one alignment cycle includes about 10 to about 20 individual DIA MS2 acquisitions.

As mentioned above, system 300 may schedule one or more alignment cycles in the acquisition schedule generated at operation 402. For example, system 300 may schedule alignment cycles to occur at periodic intervals (e.g., once every 10 seconds, once every minute, once every Nth DIA MS2 acquisition cycle, etc.). In other examples, system 300 schedules the alignment cycles to occur at times based on the expected elution time of the analytes (e.g., at times of relatively low analyte density or low analyte signal, etc.).

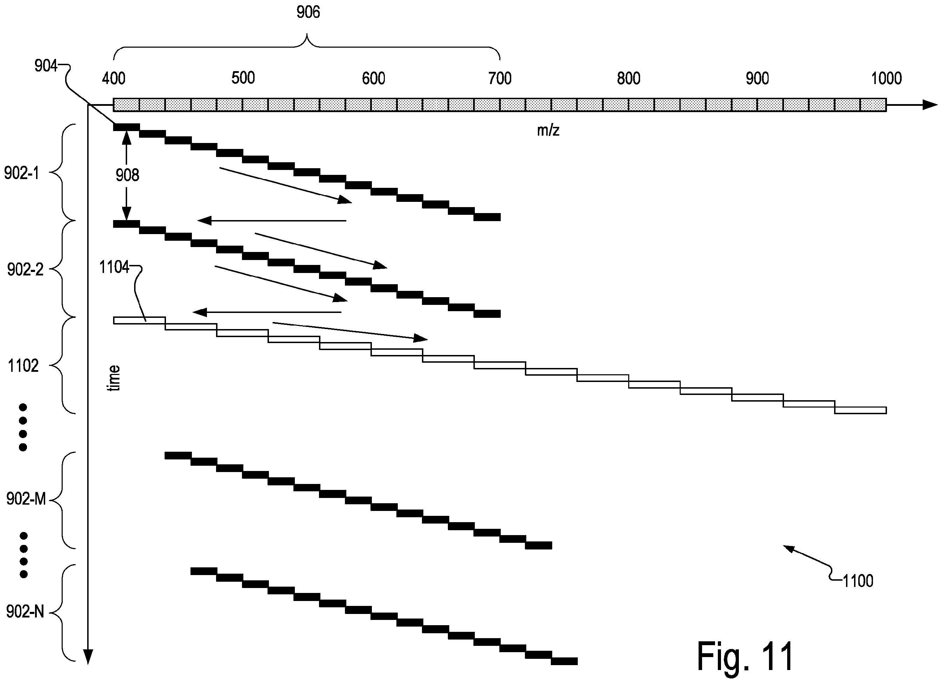

FIG. 11 shows an illustrative scheme 1100 for acquiring alignment spectra during a dynamic DIA experiment. FIG. 11 is similar to FIG. 9 except that, in FIG. 11, system 300 performs an alignment cycle 1102 after scheduled acquisition cycle 902-2. Alignment cycle 1102 is performed by advancing an isolation window 1104 across an alignment precursor m/z range that spans from 400 m/z to 1000 m/z.

Returning to FIG. 10, at operation 1004, system 300 determines a similarity of the set of alignment spectra relative to a set of reference spectra. The reference spectra represent an expected elution profile (e.g., elution times) of the analytes included in the sample. The reference spectra may be acquired in any suitable way. In some examples, the reference spectra are acquired by performing a reference run prior to performing the dynamic DIA experiment. In the reference run, a set of reference spectra are acquired in a reference cycle by DIA MS2 by isolating precursor ions using an isolation window successively positioned throughout the alignment precursor m/z range during each reference cycle. The isolation window of a reference cycle may have a relatively wide isolation width (e.g., 10 m/z, 20 m/z, etc.). In other examples, the reference spectra are acquired by inserting periodic reference cycles in a characterization analysis of the sample, such as a characterization analysis used to generate the analyte density matrix (e.g., scheme 600).

The set of reference spectra that are compared with the alignment spectra include the reference spectra acquired within a threshold period of time relative to the current time of the dynamic DIA experiment. For example, the set of reference spectra may include the reference spectra acquired within +/- N minutes of the current experimental time, where N may be, for example, 3 minutes, 5 minutes, 8 minutes, 10 minutes, etc.

The similarity of the set of alignment spectra may be determined in any suitable way. In some examples, the similarity is determined by cross-correlating the set of alignment spectra with the set of reference spectra. Any suitable cross-correlation method or algorithm may be used, including the cross-correlation method described in the following publication, which is incorporated herein by reference: Remes, P. M.; Yip, P.; MacCoss, M. J. Highly Multiplex Targeted Proteomics Enabled by Real-Time Chromatographic Alignment. Anal. Chem. 2020, 92 (17), pp. 11809-11817. In some examples, system 300 determines, based on a cross-correlation or similarity algorithm, a similarity score indicative of the degree of similarity between the set of alignment spectra and the set of reference spectra.

At operation 1006, system 300 detects, based on the determined similarity of the set of alignment spectra with the set of reference spectra, an elution time shift of the analytes. System 300 may detect the elution time shift in any suitable way, such as when the cross-correlation or similarity score satisfies an elution time shift criteria.

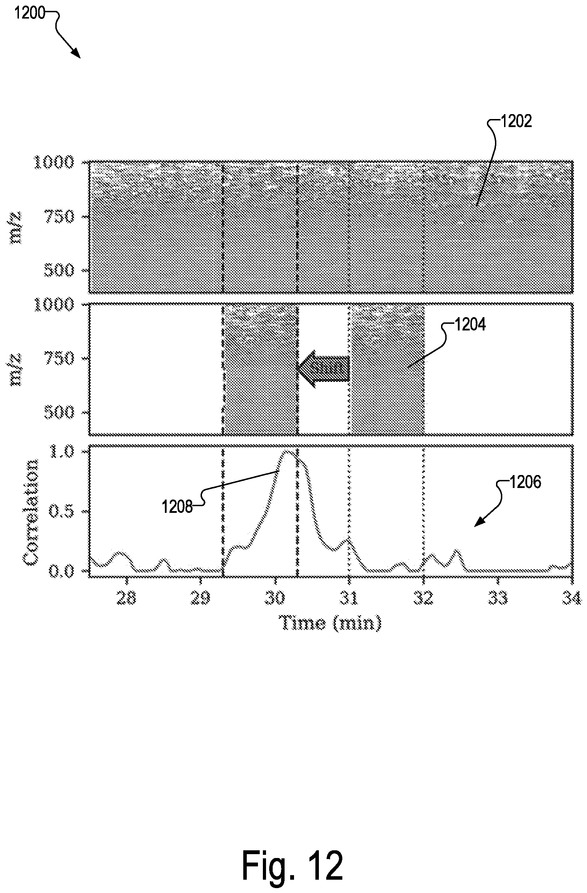

FIG. 12 shows an illustrative technique 1200 for detecting elution time shift by cross-correlating a set of alignment spectra with a set of reference spectra. System 300 obtains a set 1202 of reference spectra previously acquired or generated, as described above. At the current experimental elution time of 32 minutes, system 300 separately cross-correlates each alignment spectrum included in a set 1204 of alignment spectra with the set 1202 of reference spectra in the region of the reference spectra surrounding the current experimental elution time (e.g., +/- 5 minutes). System 300 averages the cross-correlation of each alignment spectrum to obtain an overall correlation, and the overall correlation may be characterized over time to obtain a correlation profile 1206. The elution time shift, if any, may be detected by the presence of a peak of the correlation profile 1206. As shown in FIG. 12, system 300 detects the elution time shift based the peak 1208 of the correlation profile 1206. As shown, system 300 may determine that the current elution time of 32 minutes corresponds to the expected elution time of approximately 30.4 minutes in the set of reference spectra.

In the example of FIG. 11, system 300 detects the elution time shift by comparing a set of MS2 alignment spectra acquired in real-time during a dynamic DIA experiment (e.g., during a scheduled acquisition) with a set of MS2 reference spectra. In an alternative example, MS1 reference spectra and an MS1 alignment spectrum may be used in place of MS2 reference spectra and MS2 alignment spectra. In these examples, system 300 detects the elution time shift by comparing (e.g., cross-correlating) an MS1 alignment spectrum acquired in real-time during the dynamic DIA experiment with MS1 reference spectra acquired during a reference run. However, the signal-to-noise ratio of the correlation of MS1 alignment spectrum with the MS1 reference spectra is generally lower than for a set of multiple MS2 spectra.

In another example of detecting elution time shift, no additional alignment cycles are scheduled or run during the dynamic DIA experiment. Instead, the MS2 spectra acquired by the acquisition cycles provide the information needed to detect elution time shift and perform elution time alignment. In these examples, system 300 determines a similarity of a set of MS2 spectra acquired in real-time during a dynamic DIA experiment to a set of MS2 reference spectra acquired in a reference run prior to the scheduled MS2 analysis. System 300 may determine the similarity of the MS2 spectra in any suitable way, including any manner described above for alignment spectra (e.g., by cross-correlation). Using the MS2 spectra instead of alignment spectra poses some additional complexity in terms of analysis, since the set of MS2 spectra may not correspond in time to the set of reference spectra if there is a significant elution time shift. However, this issue is surmountable by comparing only the set of MS2 spectra and reference spectra that are common in time. This may be done, for example, by performing the elution time alignment process relatively frequently and/or by spectral peak matching.

Returning again to FIG. 4, at operation 408, system 300 adjusts the acquisition schedule based on the detected elution time shift, thereby performing an elution time alignment of the acquisition schedule. System 300 may adjust the acquisition schedule by determining the amount and direction of elution time shift and adjusting the dynamic precursor m/z range for one or more subsequent acquisition cycles based on the analyte density matrix and the amount and direction of the elution time shift. For example, referring again to FIG. 11, after performing alignment cycle 1102 and detecting the elution time shift as shown in FIG. 12, system 300 may adjust the upper and lower bounds of dynamic precursor m/z range 906 for acquisition cycle 902-M and acquisition cycle 902-N from 440 m/z and 460 m/z, respectively, to 420 m/z and 440 m/z, respectively. In this way, the dynamic precursor m/z range 906 may be positioned along the m/z domain to optimize the number of analytes that are analyzed.

After adjusting the acquisition schedule, system 300 may direct the mass spectrometer to acquire, based on the adjusted acquisition schedule, MS2 spectra of product ions derived from the analytes. Method 400 may be repeated as desired to monitor for elution time shift and perform elution time adjustment of the acquisition schedule.

Various modifications may be made to the examples and embodiments described herein. For example, an acquisition schedule may be generated or predetermined (prior to performing method 400) for a particular known or pre-characterized sample. Accordingly, operation 402 in method 400 may be omitted. In other examples, the acquisition schedule may additionally or alternatively be generated based on a list of target analytes.

In certain embodiments, one or more of the systems, components, and/or processes described herein may be implemented and/or performed by one or more appropriately configured computing devices. To this end, one or more of the systems and/or components described above may include or be implemented by any computer hardware and/or computer-implemented instructions (e.g., software) embodied on at least one non-transitory computer-readable medium configured to perform one or more of the processes described herein. In particular, system components may be implemented on one physical computing device or may be implemented on more than one physical computing device. Accordingly, system components may include any number of computing devices, and may employ any of a number of computer operating systems.

In certain embodiments, one or more of the processes described herein may be implemented at least in part as instructions embodied in a non-transitory computer-readable medium and executable by one or more computing devices. In general, a processor (e.g., a microprocessor) receives instructions, from a non-transitory computer-readable medium, (e.g., a memory, etc.), and executes those instructions, thereby performing one or more processes, including one or more of the processes described herein. Such instructions may be stored and/or transmitted using any of a variety of known computer-readable media.

A computer-readable medium (also referred to as a processor-readable medium) includes any non-transitory medium that participates in providing data (e.g., instructions) that may be read by a computer (e.g., by a processor of a computer). Such a medium may take many forms, including, but not limited to, non-volatile media, and/or volatile media. Non-volatile media may include, for example, optical or magnetic disks and other persistent memory. Volatile media may include, for example, dynamic random access memory ("DRAM"), which typically constitutes a main memory. Common forms of computer-readable media include, for example, a disk, hard disk, magnetic tape, any other magnetic medium, a compact disc read-only memory ("CD-ROM"), a digital video disc ("DVD"), any other optical medium, random access memory ("RAM"), programmable read-only memory ("PROM"), electrically erasable programmable read-only memory ("EPROM"), FLASH-EEPROM, any other memory chip or cartridge, or any other tangible medium from which a computer can read.

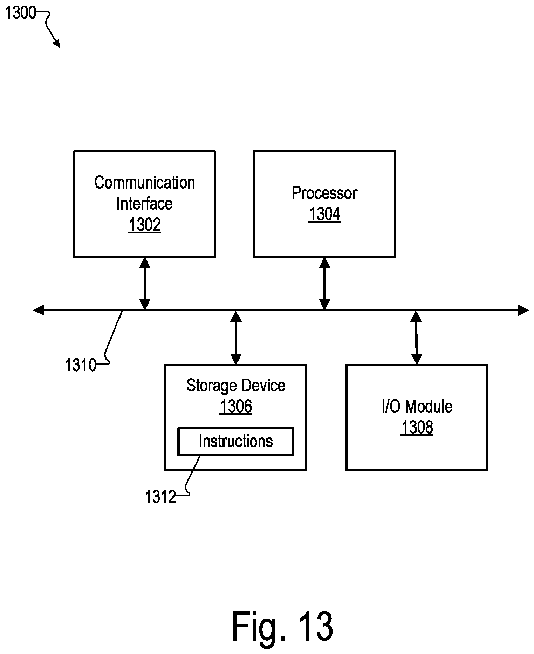

FIG. 13 shows an illustrative computing device 1300 that may be specifically configured to perform one or more of the processes described herein. As shown in FIG. 13, computing device 1300 may include a communication interface 1302, a processor 1304, a storage device 1306, and an input/output ("I/O") module 1308 communicatively connected one to another via a communication infrastructure 1310. While an illustrative computing device 1300 is shown in FIG. 13, the components illustrated in FIG. 13 are not intended to be limiting. Additional or alternative components may be used in other embodiments. Components of computing device 1300 shown in FIG. 13 will now be described in additional detail.

Communication interface 1302 may be configured to communicate with one or more computing devices. Examples of communication interface 1302 include, without limitation, a wired network interface (such as a network interface card), a wireless network interface (such as a wireless network interface card), a modem, an audio/video connection, and any other suitable interface.

Processor 1304 generally represents any type or form of processing unit capable of processing data and/or interpreting, executing, and/or directing execution of one or more of the instructions, processes, and/or operations described herein. Processor 1304 may perform operations by executing computer-executable instructions 1312 (e.g., an application, software, code, and/or other executable data instance) stored in storage device 1306.

Storage device 1306 may include one or more data storage media, devices, or configurations and may employ any type, form, and combination of data storage media and/or device. For example, storage device 1306 may include, but is not limited to, any combination of the non-volatile media and/or volatile media described herein. Electronic data, including data described herein, may be temporarily and/or permanently stored in storage device 1306. For example, data representative of computer-executable instructions 1312 configured to direct processor 1304 to perform any of the operations described herein may be stored within storage device 1306. In some examples, data may be arranged in one or more databases residing within storage device 1306.

I/O module 1308 may include one or more I/O modules configured to receive user input and provide user output. One or more I/O modules may be used to receive input for a single virtual experience. I/O module 1308 may include any hardware, firmware, software, or combination thereof supportive of input and output capabilities. For example, I/O module 1308 may include hardware and/or software for capturing user input, including, but not limited to, a keyboard or keypad, a touchscreen component (e.g., touchscreen display), a receiver (e.g., an RF or infrared receiver), motion sensors, and/or one or more input buttons.

I/O module 1308 may include one or more devices for presenting output to a user, including, but not limited to, a graphics engine, a display (e.g., a display screen), one or more output drivers (e.g., display drivers), one or more audio speakers, and one or more audio drivers. In certain embodiments, I/O module 1308 is configured to provide graphical data to a display for presentation to a user. The graphical data may be representative of one or more graphical user interfaces and/or any other graphical content as may serve a particular implementation.

In some examples, any of the systems, computing devices, and/or other components described herein may be implemented by computing device 1300. For example, memory 302 may be implemented by storage device 1306, and processor 304 may be implemented by processor 1304.

It will be recognized by those of ordinary skill in the art that while, in the preceding description, various illustrative embodiments have been described with reference to the accompanying drawings. It will, however, be evident that various modifications and changes may be made thereto, and additional embodiments may be implemented, without departing from the scope of the invention as set forth in the claims that follow. For example, certain features of one embodiment described herein may be combined with or substituted for features of another embodiment described herein. The description and drawings are accordingly to be regarded in an illustrative rather than a restrictive sense.

## Claims

1. A non-transitory computer-readable medium storing instructions that, when executed, direct at least one processor of a computing device for mass spectrometry to:
direct a mass spectrometer 104 to acquire 404, based on an acquisition schedule that schedules a plurality of acquisition cycles, MS2 spectra of product ions derived from analytes included in a sample as the analytes elute from a separation system, wherein:
the acquisition schedule specifies, for each acquisition cycle included in the plurality of acquisition cycles, a dynamic precursor m/z range based on an expected elution time of the analytes; and
the product ions are produced from precursor ions isolated using an isolation window successively positioned throughout the dynamic precursor m/z range during each acquisition cycle;
detect 406 an elution time shift in the elution time of the analytes as the analytes elute from the separation system; and
adjust 408 the acquisition schedule based on the detected elution time shift.

2. The computer-readable medium of claim 1, wherein detecting the elution time shift comprises:
directing the mass spectrometer to acquire, during acquisition of the MS2 spectra based on the acquisition schedule, a set of alignment spectra;
determining a similarity of the set of alignment spectra to a set of reference spectra surrounding a current elution time, the reference spectra representing an expected elution profile of the analytes; and
detecting, based on the determined similarity of the set of alignment spectra, the elution time shift.

3. The computer-readable medium of claim 2, wherein the determining the similarity of the set of alignment spectra to the set of reference spectra comprises cross-correlating the set of alignment spectra with the set of reference spectra.

4. The computer-readable medium of claim 2, wherein the alignment spectra and the reference spectra comprise MS1 spectra.

5. The computer-readable medium of claim 2, wherein the alignment spectra and the reference spectra comprise MS2 spectra.

6. The computer-readable medium of claim 2, wherein the acquisition schedule includes one or more alignment cycles for acquisition of the set of alignment spectra.

7. The computer-readable medium of claim 1, wherein the detecting the elution time shift comprises:
determining a similarity of a set of the MS2 spectra to a set of reference spectra, the reference spectra representing an expected elution profile of the analytes; and
detecting, based on the determined similarity of the set of MS2 spectra, the elution time shift.

8. The computer-readable medium of claim 1, wherein a position of the dynamic precursor m/z range within the acquisition schedule as a function of elution time is configured to optimize a quantity of the analytes represented in the MS2 spectra.

9. The computer-readable medium of claim 1, wherein the instructions, when executed, further direct the at least one processor to determine an isolation width of the isolation window based on a chromatogram library associated with the sample and a minimum number of quantifiable transitions.

10. The computer-readable medium of claim 1, wherein the instructions, when executed, further direct the at least one processor to direct the mass spectrometer to acquire MS2 spectra of the product ions based on the adjusted acquisition schedule.

11. The computer-readable medium of any of claims 1-10, wherein the instructions, when executed, further direct the at least one processor to generate 402 the acquisition schedule based on an analyte density matrix associated with the sample, wherein the analyte density matrix represents an expected quantity of the analytes as a function of m/z and an expected elution time of the analytes.

12. The computer-readable medium of claim 11, wherein the instructions, when executed, further direct the at least one processor to generate, prior to generating the acquisition schedule, the analyte density matrix based on a DIA MS2 characterization analysis of the sample, the characterization analysis covering a characterization m/z range of interest.

13. The computer-readable medium of claim 12, wherein the characterization analysis comprises executing the instructions to:
direct the mass spectrometer to acquire, over time for each injection included in a set of injections of the sample to the separation system, MS2 characterization spectra of ions derived from the analytes by successively positioning an isolation window within a subset of a characterization m/z range of interest;
generate, based on the MS2 characterization spectra and a spectral library, a chromatogram library associated with the sample; and
generate, based on the chromatogram library associated with the sample, the analyte density matrix.
